(19) Europäisches Patentamt
European Patent Office
Office européen des brevets

(11) Veröffentlichungsnummer : **0 120 297**
**B1**

(12) **EUROPÄISCHE PATENTSCHRIFT**

(45) Veröffentlichungstag der Patentschrift : **28.05.86**

(51) Int. Cl.[4] : **C 07 D309/26**

(21) Anmeldenummer : **84101879.9**

(22) Anmeldetag : **23.02.84**

(54) **Verfahren zur Herstellung von 3-Formyl-5,6-dihydro-2H-pyran.**

(30) Priorität : **04.03.83 DE 3307635**

(43) Veröffentlichungstag der Anmeldung : **03.10.84 Patentblatt 84/40**

(45) Bekanntmachung des Hinweises auf die Patenterteilung : **28.05.86 Patentblatt 86/22**

(84) Benannte Vertragsstaaten : **BE CH DE FR GB IT LI NL**

(56) Entgegenhaltungen :
**US-A- 2 514 156**
**BEILSTEINS HANDBUCH DER ORGANISCHEN CHEMIE, 4. Auflage, 3. und 4. Ergänzungswerk, Band 17, Teil 5, 1975 SPRINGER-VERLAG, Berlin-Heidelberg-New York Seite 4306**
**CHEMICAL ABSTRACTS, Vol. 92, No. 17, 28. April 1980, Columbus, Ohio, USA UNTERHALT, BERNARD; GHORI, MAHMOOD "Oximes as artificial sweeteners. Part 23. Unsaturated oximes" Seite 570, Spalte 1, Abstract-No. 146 55a**
**CHEMICAL ABSTRACTS, Vol. 50, No. 16, 25. August 1956, Columbus, Ohio, USA R.H. HALL "Derivatives of acraldehyde dimer" Spalte 11 335, Abstract-No. c**
**CHEMICAL ABSTRACTS, Vol. 51, No. 1, 10. Jänner 1957, Columbus, Ohio, USA GERHARD DUMAS, PAUL RUMPF "A new proof of structure of the cyclic dimers of alpha-beta-ethylenic hydrocarbons" Spalte 386, Abstract-No. h**
**CHEMICAL ABSTRACTS, Vol. 65, No. 12, 5. Dezember 1966, Columbus, Ohio, USA AKIRA MISONO, TETSUO OSA, TAKAMICHI YAMAGISHI "The hydrodimerization of acrolein by electroreduction" Spalte 18 450, Abstract-No. d**

(73) Patentinhaber : **BASF Aktiengesellschaft**
**Carl-Bosch-Strasse 38**
**D-6700 Ludwigshafen (DE)**

(72) Erfinder : **Eckhardt, Heinz, Dr.**
**Ruedigerstrasse 7**
**D-6700 Ludwigshafen (DE)**
Erfinder : **Halbritter, Klaus, Dr.**
**Schwarzwaldstrasse 19**
**D-6800 Mannheim 1 (DE)**
Erfinder : **Goetz, Norbert, Dr.**
**Schoefferstrasse 25**
**D-6520 Worms 1 (DE)**
Erfinder : **Heilen, Gerd, Dr.**
**Schifferstadter Strasse 1b**
**D-6720 Speyer (DE)**

**EP 0 120 297 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung von 3-Formyl-5,6-dihydro-2H-pyran durch Umsetzung von Acrolein in Gegenwart von Wasser, Säuren und Halogenkohlenwasserstoffen mit 1 bis 6 Kohlenstoffatomen und 1 bis 6 Halogenatomen als Lösungsmittel bei einer Temperatur zwischen 60 und 150 °C.

In der US-PS 2 514 156 ist eine Umsetzung von Acrolein in Gegenwart von wäßriger Salzsäure und Toluol bei bevorzugt 40 bis 70 °C (Beispiel 1) mit einer Ausbeute von 32 % 3-Formyl-5,6-dihydro-2H-pyran beschrieben. Es wird ausdrücklich festgestellt, daß die Anwendung höherer Reaktionstemperaturen zu schlechteren Ergebnissen (Spalte 3, Zeilen 16 bis 33) führt und die Verwendung inerter Lösungsmittel das Ergebnis der Umsetzung nicht beeinflußt, sondern lediglich die Regulierung der Reaktionstemperatur erleichtert (Spalte 3, Zeilen 44 bis 51). Neben Toluol wird noch Benzol genannt.

In C.W. Smith, Acrolein (Heidelberg, 1975), wird eine Umsetzung von Acrolein bei 170 °C ohne Säurezusatz in Gegenwart von Benzol, Furan oder Ethylether beschrieben, wobei in 40 bis 45 %iger Ausbeute 3,4-Dihydro-2(H)-pyran-2-carboxaldehyd entsteht (loc. cit., Seite 168, Absatz 1). Es wird darauf hingewiesen, daß die organischen Lösungsmittel nur verwendet werden, um eine Verkohlung zu verhindern, und gute Ausbeuten in Abwesenheit von Lösungsmitteln erhalten wurden. Weiterhin ist bekannt (loc. cit., Seite 199 oben ; Seite 201, 3. Absatz), daß bei Anlagerung von ungesättigten Verbindungen an Acrolein höhere Temperaturen, nämlich 150 bis 200 °C, erforderlich sind. Die Veröffentlichung gibt weiterhin (loc. cit., Seite 140) eine Reaktion von Acrolein bei 70 °C in Gegenwart von wäßriger Phosphorsäure und von Benzol zur Herstellung von 3-Formyl-5,6-dihydro-2H-pyran an. Unter diesen Bedingungen ist die Umsetzung unvollständig, denn die Benzolphase muß mehrfach abgetrennt und destilliert werden, wobei bei jedem Durchgang das unumgesetzte Acrolein zusammen mit dem benzol in das Reaktionsgefäß zurückgeführt werden muß. Man erzielt so in einer Gesamtreaktionszeit von 10 Stunden eine Gesamtausbeute von 58 %. Aufgrund der toxikologischen Eigenschaften von Benzol und Acrolein ist ein solches langfristiges Manipulieren mit diesen Stoffen, insbesondere im Technischen Maßstab im Hinblick auf einfachen und wirtschaftlichen Betrieb, Betriebssicherheit und Umweltschutz nachteilig.

Es wurde nun gefunden, daß man 3-Formyl-5,6-dihydro-2H-pyran der Formel I

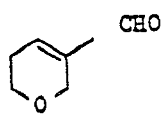

(I)

durch Umsetzung von Acrolein in Gegenwart von Wasser, Säuren und organischen Lösungsmitteln bei erhöhter Temperatur vorteilhaft erhält, wenn man die Umsetzung in Gegenwart von Halogenkohlenwasserstoffen mit 1 bis 6 Kohlenstoffatomen und 1 bis 6 Halogenatomen als Lösungsmittel bei einer Temperatur zwischen 60 und 150 °C durchführt.

Die Umsetzung läßt sich durch die folgenden Formeln wiedergeben :

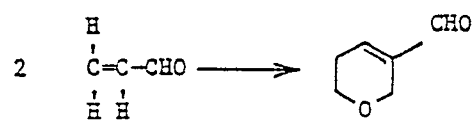

Im Hinblick auf die bekannten Verfahren liefert das Verfahren nach der Erfindung auf einfacherem und wirtschaftlicherem Wege 3-Formyl-5,6-dihydro-2H-pyran in besserer Raum-Zeit-Ausbeute und meist besserer Ausbeute und Reinheit Längere Reaktionszeiten und zahlreiche Aufarbeitungsschritte unter Verwendung von Benzol werden vermieden ; das erfindungsgemäße Verfahren ist daher mit Bezug auf Schutz des betriebspersonals, Betriebssicherheit, Einsparung von Aufarbeitungsapparaturen und entsprechenden Betriebskontrollen sowie Umweltschutz vorteilhafter.

Alle diese vorteilhaften Ergebnisse des erfindungsgemäßen Verfahrens sind im Hinblick auf den Stand der Technik überraschend. Man konnte nicht erwarten, daß durch die Verwendung eines bestimmten Lösungsmittels die Ausbeute an Endstoff in erheblichem Maße beeinflußt wird. Ebenfalls ist überraschend, daß nicht Ether bzw. aromatische Kohlenwasserstoffe, sondern gerade die Gruppe der erfindungsgemäßen Halogenkohlenwasserstoffe vorteilhafte Ergebnisse liefern. Ebenfalls konnte mit Bezug auf Smith (loc. cit.) nicht erwartet werden, daß gerade der erfindungsgemäße Temperaturbereich für die Umsetzung vorteilhaft ist, und mit Bezug auf die US-PS war es überraschend, daß auch Temperaturen über 70 °C vorteilhafte Ergebnisse liefern.

Die Umsetzung wird bei einer Temperatur zwischen 60 und 150 °C, bevorzugt zwischen 60 und 120 °C, insbesondere zwischen 70 und 120 °C, unter Überdruck, unter Druck oder zweckmäßig drucklos, kontinuierlich oder diskontinuierlich durchgeführt.

Wasser wird zweckmäßig in einer Menge von 100 bis 1 000, vorzugsweise 100 bis 500 Gew.%, bezogen auf die Gewichtsmenge Acrolein, verwendet. Wasser und Säure können getrennt voneinander oder als

Gemisch dem Ausgangsgemisch zugesetzt werden. Zweckmäßig bildet man mit einem entsprechenden Anteil des Wassers eine wäßrige Säurelösung und gibt den restlichen Wasseranteil getrennt dem Ausgangsgemisch zu.

Man verwendet als organische Lösungsmittel in der Regel cycloaliphatische, aromatische oder vorzugsweise aliphatische, ungesättigte oder zweckmäßig gesättigte Halogenkohlenwasserstoffe, vorteilhaft Bromkohlenwasserstoffe oder bevorzugt Chlorkohlenwasserstoffe, mit 1 bis 6, bevorzugt 1 bis 4 Kohlenstoffatomen, und 1 bis 6, bevorzugt 1 bis 4 Halogenatomen. Als Halogenkohlenwasserstoffe kommen in Frage : Amylchlorid, Cyclohexylchlorid, Dichlorbutane, Isopropylbromid, n-Propylbromid, Butylbromid, Ethyliodid, Propyliodid, Pentachlorethan, 1,1-Dichlorethan, n-Propylchlorid, 1,2-cis-Dichlorethylen, n-Butylchlorid, 2-, 3- oder iso-Butylchlorid, Fluorbenzol, Brombenzol, Jodbenzol, o-, p- und m-Dichlorbenzol, o-, p-, m-Dibrombenzol, 1,2,4-Trichlorbenzol, 1,4-Dibrombutan ; vorteilhaft Tri-, Tetrachlormethan, 1,1,1,2-Tetrachlor- und 1,1,2,2-Tetrachlorethan, 1,1-, 1,2-, 1,3- und 2,2-Dichlorpropan, Trichlorpropan, Tetrachlorethylen, Chlorbenzol ; bevorzugt Dichlormethan, 1,2-Dichlorethan, 1,1,1- und 1,1,2-Trichlorethan und Trichlorethylen ; und entsprechende Gemische. Zweckmäßig verwendet man das Lösungsmittel in einer Menge von mindestens 10 Vol.%, zweckmäßig von 10 bis 200, vorteilhaft 10 bis 100 vol.%, vorzugsweise von 20 bis 80 Gew.%, bezogen auf die Volumenmenge Wasser.

Die Umsetzung wird in Gegenwart von Säuren als Katalysator, vorteilhaft mit einer Menge von 0,01 bis 10, insbesondere von 0,1 bis 3 Äquivalenten Säure, bezogen auf 1 Mol Acrolein, durchgeführt. Es können organische oder insbesondere anorganische Säuren verwendet werden. Anstelle einbasischer Säuren können auch äquivalente Mengen mehrbasischer Säuren zur Anwendung gelangen. Zweckmäßig werden Säuren mit einem $P_{K_s}$-Wert von höchstens 4, vorteilhaft von 1 bis 3, insbesondere 1,5 bis 2,5 eingesetzt. Bevorzugt sind starke Mineralsäuren. Beispielsweise sind folgende Säuren geeignet : Chlorwasserstoff, Bromwasserstoff, Jodwasserstoff, Schwefelsäure, Phosphorsäure, Salpetersäure : Sulfonsäuren wie Benzol- und p-Toluolsulfonsäure ; Bor enthaltende Säuren wie Borsäure. Borfluorwasserstoffsäure ; aliphatische Carbonsäuren wie Chloressigsäure, Dichloressigsäure, Trichloressigsäure, Oxalsäure, o- und p-Chlorbenzoesäure oder entsprechende Gemische. Die Säuren können in konzentrierter Form, im Gemisch miteinander und/oder zweckmäßig mit Wasser, angewendet werden. Bei verdünnten, wäßrigen Säuren sind 2 bis 40 gew.%ige Säuren, z. B. 2 bis 25 gew.%ige Salzsäure, 2 bis 25 gew.%ige Schwefelsäure, 10 bis 40 gew.%ige Phosphorsäure oder 2 bis 10 gew.%ige Salpetersäure, vorteilhaft. Die Acidität der Säure sollte mindestens der einer 2 gew.%igen, wäßrigen Schwefelsäure entsprechen, vorteilhaft der Acidität von wäßriger, 3 gew.%iger bis 20 gew.%iger, bevorzugt 5 gew.%iger bis 10 gew.%iger Schwefelsäure oder von wäßriger, 10 gew.%iger Phosphorsäure. Bevorzugt sind Salzsäure, p-Toluolsulfonsäure, Monochloressigsäure, Di-,Trichloressigsäure, Oxalsäure, insbesondere Schwefelsäure oder Phosphorsäure.

Die Reaktion kann wie folgt durchgeführt werden : Ein Gemisch von Acrolein, Säure, Wasser und Halogenkohlenwasserstoff wird während der Reaktionszeit bei der Reaktionstemperatur gehalten. Zweckmäßig werden wäßrige Säuren und das Lösungsmittel im Reaktionsgefäß vorgelegt und bei der Reaktionstemperatur in einer Zeit von vorteilhaft 15 bis 150 Minuten Acrolein oder eine Mischung aus Acrolein und dem Lösungsmittel zugegeben. Die Reaktionszeit beträgt zweckmäßig 1 bis 15 Stunden, vorteilhaft 2 bis 6 Stunden. Aus dem Reaktionsgemisch wird dann der Endstoff in üblicher Weise, z. B. Abkühlen des zweiphasigen Reaktionsgemisches, Abtrennung der organischen Phase und Destillation dieser Phase, isoliert. Die wäßrige Phase wird vorteilhaft in einem weiteren Ansatz anstelle neuer wäßrigen Säure wiederverwendet. Ist vorgesehen, die Umsetzung in einer Druckapparatur durchzuführen, ist es auch zweckmäßig, das Acrolein und den Halogenkohlenwasserstoff vorzulegen, die wäßrige Säure hinzuzugeben und dieses Gemisch während der vorgenannten Zeit der Reaktionstemperatur zu halten.

Das nach dem Verfahren der Erfindung herstellbare 3-Formyl-5,6-dihydro-2H-pyran ist ein wertvoller Ausgangsstoff für die Herstellung von Farbstoffen und Pflanzenschutzmitteln.

Bezüglich der Verwendung wird auf die vorgenannten Veröffentlichungen verwiesen.

## Beispiel 1

a) 564 g Wasser, 36 g Schwefelsäure (ber. 100 %) und 400 ml 1,1,2-Trichlorethan wurden zum Sieden erhitzt. Man tropfte innerhalb von 1 Stunde unter Rühren 125 g Acrolein bei 85 °C zu und erhitzte das Gemisch weitere 6 Stunden unter Rückfluß (87 °C). Die untere, organische Phase des Reaktionsgemischs wurde abgetrennt und die obere noch einmal mit 100 ml Trichlorethan extrahiert. Beide organischen Phasen wurden destilliert. Man erhielt 66 g (53 % der Theorie) 3-Formyl-5,6-dihydro-2H-pyran vom Kp. 96 °C (38 mbar). Reinheit 99,3 %.

b) Die wäßrige phase wurde mit 400 ml 1,1,2-Trichlorethan versetzt und die Umsetzung 1a) mit 125 g Acrolein wiederholt. Man erhielt 75 g (60 % der Theorie) 3-Formyl-5,6-dihydro-2H-pyran vom Kp. 96 °C (38 mbar). Reinheit 99,3 %.

## Beispiel 2

Man verfuhr wie in Beispiel 1, werwendete jedoch 540 g Wasser und 60 g Schwefelsäure (ber. 100 %).

Bei der ersten Umsetzung (1a) erhielt man 72 g (58 % der Theorie) und bei der Folgeumsetzung (1b) 78 g (63 % der Theorie) 3-Formyl-5,6-dihydro-2H-pyran vom Kp. 93 °C (30 mbar). Reinheit 99,3 %.

Beispiel 3

Man verfuhr wie in Beispiel 1, verwendete jedoch 388 g Wasser und 212 g wäßrige ortho-Phosphorsäure (85 Gew.%). Bei der ersten Umsetzung (1a) erhielt man 79 g (63 % der Theorie) und bei der Folgeumsetzung (1b) 91 g (73 % der Theorie) 3-Formyl-5,6-dihydro-2H-pyran vom Kp. 93 °C (30 mbar). Reinheit 99,3 %.

Beispiel 4

a) 560 g Wasser, 744 g Dichlormethan, 248 g Acrolein und 136 g wäßrige ortho-Phosphorsäure (85 Gew.%) wurden im Autoklaven 12 Stunden bei 80 °C gehalten. Man trennte die Phasen und extrahierte die obere, wäßrige Phase mit 400 ml Dichlormethan. Die Destillation der organischen Phase lieferte 112 g (45 % der Theorie) 3-Formyl-5,6-dihydro-2H-pyran vom Kp. 93 °C (30 mbar). Reinheit 99,3 %.

b) Die wäßrige Phase wurde zusammen mit 248 g frischem Acrolein und 744 g Dichlormethan analog (1a) umgesetzt. Man erhielt 131 g (53 % der Theorie) 3-Formyl-5,6-dihydro-2H-pyran vom Kp. 93 °C (30 mbar). Reinheit 99,3 %.

Beispiel 5

Man verfuhr wie in Beispiel 3, verwendete jedoch 200 g Acrolein. Bei der ersten Umsetzung (3a) erhielt man 122 g (61 % der Theorie) und bei der Folgeumsetzung (3b) 138 g (69 % der Theorie) 3-Formyl-5,6-dihydro-2H-pyran vom Kp. 93 °C (30 mbar). Reinheit 99,2 %.

Beispiel 6

Man verfuhr wie in Beispiel 3, verwendete jedoch 400 ml Trichlorethylen. Bei der ersten Umsetzung (3a) erhielt man 76 g (61 % der Theorie) und bei der Folgeumsetzung (1b) 80 g (64 % der Theorie) 3-Formyl-5,6-dihydro-2H-pyran vom Kp. 93 °C (30 mbar). Reinheit 99,2 %.

Beispiel 7

Man verfuhr wie in Beispiel 4, verwendete jedoch 700 ml 1,1,1-Trichlorethan. Bei der ersten Umsetzung (4a) erhielt man 126 g (51 % der Theorie) und bei der Folgeumsetzung (4b) 149 g (60 % der Theorie) 3-Formyl-5,6-dihydro-2H-pyran vom Kp. 93 °C (30 mbar). Reinheit 99,2 %.

Beispiel 8 (Vergleichsbeispiel)

a) 300 g Wasser, 100 g Schwefelsäure (ber. 100 %) und 400 ml Toluol wurden zum Sieden erhitzt. Man tropfte innerhalb von 2 Stunden unter Rühren 100 g Acrolein bei 85 °C zu und erhitzte das Gemisch weitere 4 Stunden unter Rückfluß (86 °C). Die obere, organische Phase wurde abgetrennt und die untere noch einmal mit 100 ml Toluol extrahiert. Beide Toluolphasen wurden destilliert. Man erhielt 30 g (30 % der Theorie) 3-Formyl-5,6-dihydro-2H-pyran vom Kp. 95 °C (35 mbar). Reinheit 99,2 %.

b) Die wäßrige Phase wurde mit 400 ml frischem Toluol versetzt und erneut analog (1a) mit 100 g Acrolein umgesetzt. Man erhielt 45 g (45 % der Theorie) 3-Formyl-5,6-dihydro-2H-pyran vom Kp. 96 °C (38 mbar). Reinheit 99,2 %.

c) Eine dritte Umsetzung analog (1b) lieferte keine weitere Steigerung der Ausbeute.

**Patentanspruch**

Verfahren zur Herstellung von 3-Formyl-5,6-dihydro-2H-pyran der Formel I

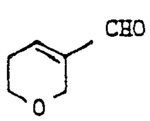

(I)

durch Umsetzung von Acrolein in Gegenwart von Wasser, Säuren und organischen Lösungsmitteln bei erhöhter Temperatur, dadurch gekennzeichnet, daß man die Umsetzung in Gegenwart von Halogenkohlenwasserstoffen mit 1 bis 6 Kohlenstoffatomen und 1 bis 6 Halogenatomen als Lösungsmitteln bei einer Temperatur zwischen 60 und 150 °C durchführt.

**Claim**

A process for the preparation of 3-formyl-5,6-dihydro-2H-pyran of the formula I

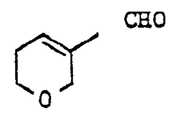

by reacting acrolein in the presence of water, acids and organic solvents at elevated temperature, wherein the reaction is carried out in the presence of halohydrocarbons of 1 to 6 carbon atoms and 1 to 6 halogen atoms, as solvents, at between 60 and 150 °C.

**Revendication**

Procédé pour la préparation de 3-formyl-5,6-dihydro-2H-pyranne de formule I

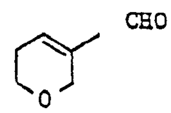

par réaction d'acroléine en présence d'eau, d'acides et de solvants organiques à température élevée, caractérisé en ce qu'on mène la réaction à une température comprise entre 60 et 150 °C en présence, en tant que solvants, d'hydrocarbures halogénés comportant 1 à 6 atomes de carbone et 1 à 6 atomes d'halogène.